# FASCICULE DE BREVET EUROPEEN

(11) **EP 0 371 889 B1**
(45) Date de publication et mention de la délivrance du brevet: **10.08.1994**
(21) Numéro de dépôt: 89420447.8
(22) Date de dépôt: 17.11.1989
(51) Int. Cl.: A61B 1/24, A63B 71/08

(54) **Dispositif à usage médical pour protéger la denture et faciliter l'introduction d'appareillage médical dans la cavité buccale**
Medizinische Vorrichtung zum Schutz der Zähne und zur Erleichterung der Einführung einer medizinischen Apparatur
Medical device for protecting the teeth and facilitating the introduction of medical equipment into the oral cavity

(30) Priorité: 24.11.1988 FR 8815636
(43) Date de publication de la demande: 06.06.1990
(73) Titulaire: Bory, Eric-Nicolas, F-69003 Lyon (FR)
(72) Inventeur: Bory, Eric-Nicolas, F-69003 Lyon (FR)
(74) Mandataire: Phélip, Bruno

(56) Documents cités:
- US-A- 3 223 085
- US-A- 3 247 844
- US-A- 3 688 406
- US-A- 4 173 219
- US-A- 4 350 154

## Description

La présente invention concerne un dispositif à usage médical, prêt à l'emploi, destiné à s'adapter aux arcades dentaires, plus spécialement à l'arcade dentaire supérieure pour faciliter l'introduction d'appareillage médical ou chirurgical dans la cavité orale, tout en protégeant les dents.

Ce dispositif comporte une gouttière rigide, en forme de fer à cheval. La partie médiane présente deux échancrures situées sur les parois interne et externe. La gouttière est solidaire d'une garniture interne en mousse. Sur le bourrelet périphérique de cette garniture, une colle est déposée.

Ce dispositif qui est mis en place ou retiré rapidement, offre de nombreux avantages.

Il facilite l'introduction du laryngoscope lors d'examens endoscopiques utilisant cet appareil, lors d'intubations trachéales par voie orale, au cours des anesthésies générales notamment, lors d'examens de la sphère aérienne supérieure, ou bien à l'occasion d'autres examens endoscopiques tels que bronchoscopies, gastroscopies, ou bien encore au moment de l'introduction d'un instrument tel que la pince de MAGILL dans la cavité buccale d'un patient anesthésié.

Il réduit le temps de mise en place de l'intubation trachéale par voie orale et plus particulièrement dans le cas difficile où se présente une zone partiellement édentée dans le groupe incisivo-canin supérieur.

Il n'est pas encombrant, ne réduit que très faiblement l'ouverture de la bouche et il reste stable pendant le travail du médecin spécialiste ou de l'anesthésiste.

Il constitue une protection efficace contre les traumatismes et les chocs provoqués par le laryngoscope ou un autre instrument médical. Il protège ainsi la denture naturelle, plus spécialement les dents dites "fragiles", et il protège aussi les éventuelles reconstitutions prothétiques fixes réalisées à l'aide de matériaux fragiles tels que céramique, matériaux composites ou résines.

Au moment de l'introduction du laryngoscope, il offre la possibilité de prendre appui délicatement et sans excès sur la partie antérieure rigide du dispositif, à condition, bien sûr, de contrôler la force en jeu.

Il évite la chute dans l'oro-pharynx, de dents fragiles expulsées parfois de leur alvéole au cours de manipulations d'instruments dans la cavité buccale.

Il assure à l'anesthésiste, tout en lui facilitant le travail, une sécurité, puisque les risques consécutifs à une intubation ne peuvent être que réduits.

Divers procédés ou dispositifs ont déjà été proposés pour protéger les dents pendant un travail médical comportant une intervention dans la cavité orale.

Certains ont proposé l'emploi de compresses de gaze ou de feuilles de plomb.

SALISBURY, P.L. et coll., 1984 (arch. otolaryngol. 110 (2) pp 106-107) ont décrit une technique nécessitant au préalable la prise d'empreinte avant la réalisation d'un dispositif individuel de protection en matière thermoplastique, dans un laboratoire de prothèse dentaire.

PODOSHIN L. et FRADIS, M., 1976 (arch. otolaryngol. 102 (7) p. 439) préconisent l'utilisation d'un dispositif fabriqué au cours de l'induction de l'anesthésie générale. Le protocole consiste à injecter une quantité donnée de monomère dans un sac cylindrique en plastique souple rempli de poudre de résine méthylmétacrylate, puis à malaxer ce sac avant de l'adapter à l'arcade dentaire supérieure.

Le document US-A-4.350.154 concerne un dispositif de protection des dents comportant une gouttière rigide qui comprend au moins une échancrure en U, ménagée dans la paroi externe de la gouttière. La garniture intérieure forme un bourrelet périphérique lors de la mise en place du dispositif dans la bouche du patient.

Le document US-A-3.223.085 concerne également un dispositif de protection des dents comportant une gouttière rigide qui comporte une échancrure en U dans la paroi externe de la gouttière.

Le dispositif, selon mon invention, prêt à l'emploi, n'exige pas de prise d'empreinte préalable. Grâce à sa conformation, il s'adapte rapidement et aisément aux arcades dentaires, selon leur forme et leur dimension. Il reste stable au cours du travail des praticiens, assure un pontage efficace au niveau d'une édentation partielle et il assure un amortissement efficace des chocs et traumatismes. Réalisé dans un matériau peu onéreux il pourrait être à usage unique, évitant, de ce fait, une éventuelle contamination entre patients.

Le dispositif, selon mon invention, comprend une gouttière, une garniture, une colle.

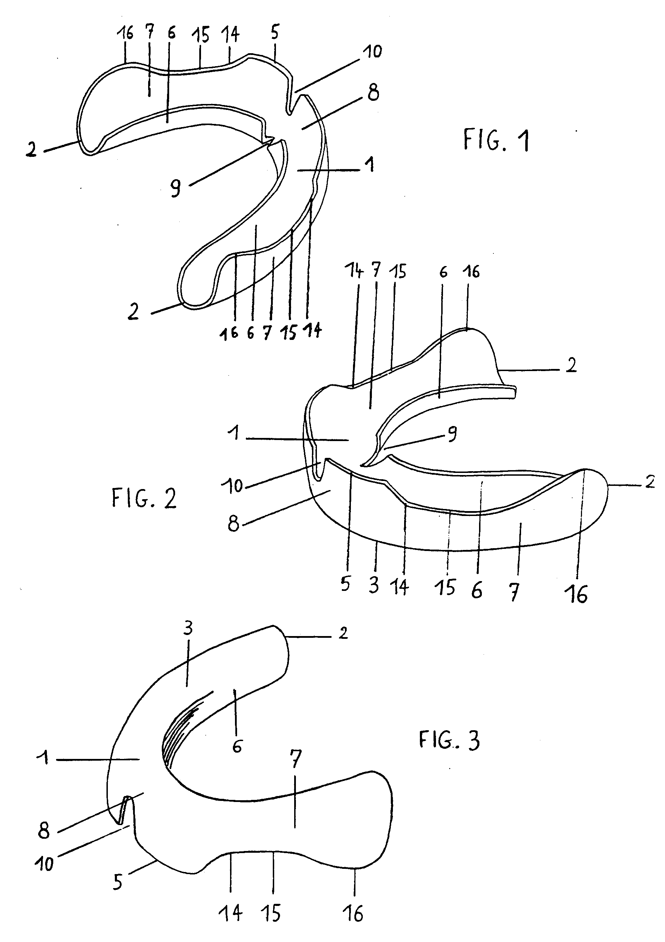

La figure 1 est une vue en perspective de la gouttière (1),

Les figures 2 et 3 sont des vues en perspective de la gouttière (1) sous des angles différents.

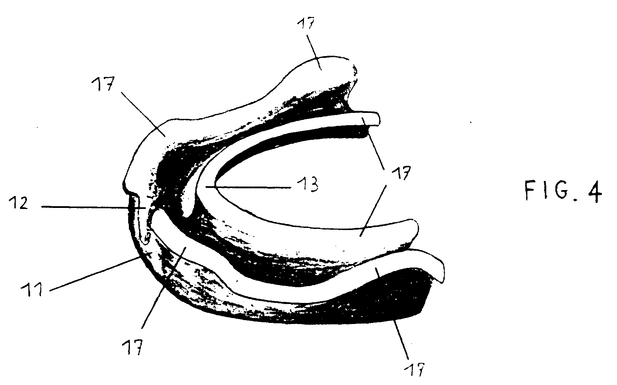

La figure 4 est une vue en perspective de la garniture (11).

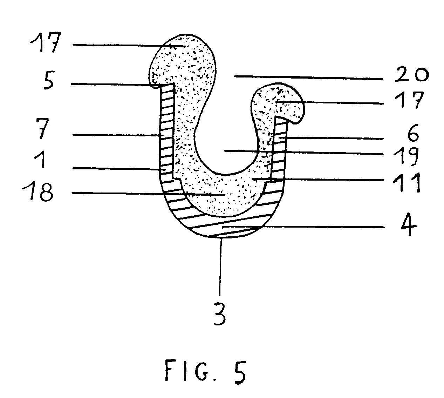

La figure 5 est une coupe transversale du dispositif.

La gouttière (1), en forme de fer à cheval recouvre l'ensemble de l'arcade dentaire, plus spécialement l'arcade dentaire supérieure, ainsi qu'une partie de la muqueuse alvéolaire adjacente, que la denture soit complète ou non.

Elle est ouverte aux deux extrémités (2) pour éviter qu'elle ne s'appuie sur le ligament ptérygomaxillaire. Sinon, celui-ci déstabiliserait la gouttière au moment de l'ouverture forcée de la bouche. Le fond (3) de la gouttière (1) est arrondi pour faciliter l'introduction, dans la cavité buccale, d' instruments utilisés par le médecin spécialiste ou par l'anesthésiste.

La gouttière (1) est réalisée dans un matériau suffisamment rigide afin qu'elle puisse tenir lieu de pontage au niveau d'une édentation partielle, afin aussi qu'elle puisse offrir la possibilité, au moment de l'introduction dans la touche d'un appareil tel que le laryngoscope, de prendre appui délicatement et sans excès sur la partie antérieure de la gouttière (8), à condition, bien sûr, de contrôler la force en jeu.

C'est pourquoi la gouttière (1), qui est rigide, est réalisée en polypropylène ou polyéthylène ou élastomères ou silicones, ou autres matériaux plastiques ou composites adéquats.

La gouttière (1) présente au niveau du fond (3) un renforcement (4) où le matériau est plus épais.

Le bord externe (5) de la gouttière (1) présente une courbure voisine de celle de la ligne mucogingivale pour éviter les brides musculaires latérales (14) et les apophyses zygomatiques (15), tout en englobant les tubérosités (16).

La hauteur des parois latérales de la gouttière diffère suivant qu'elles sont internes (6) ou externes (7). Du côté palatin (interne), la hauteur qui est de l'ordre de 10 mm, diminue progressivement et légèrement depuis la partie antérieure jusqu'à chacune des extrémités (2), cela pour permettre la pose correcte de la gouttière (1), quelle que soit la forme du palais du patient, et éviter ainsi de le blesser, Du côté vestibulaire (externe), la hauteur qui est de l'ordre de 20 mm dans la partie antérieure (8) est inégale en raison de la courbure particulière du bord externe (5) de la gouttière (1).

La gouttière (1) présente dans la partie antérieure (8) médiane, deux échancrures (9) et (10) situées en vis-à-vis respectivement en V et en U sur les parois latérales interne (6) et externe (7). L'échancrure (9) profonde de 10 mm environ assure l'articulation des deux parties symétriques de la gouttière (1), ce qui permet à celle-ci de s'adapter aux différentess arcades dentaires, selon leur forme et leur dimension. L'échancrure (10) en U, profonde de 8 à 10 mm, libère le frein de la lèvre supérieure.

La garniture (11) recouvre intérieurement la gouttière (1) et s'y adapte parfaitement.

Elle est en mousse de type polyéthylène expansé à l'azote pur et elle est obtenue pat thermo-moulage. D'autres produits pourraient être utilisés: mousse de latex, silicones, polyuréthanes, élastomères ou autres matériaux plastiques, à condition qu'ils soient souples, malléables, non friables, stérilisables, non toxiques, non irritants et non allergisants pour les muqueuses, .

Elle (11) présente, au niveau du frein médian de la lèvre supérieure, une échancrure (12) semblable à celle de la gouttière (1); par contre, elle n'a pas d'échancrure dans la partie palatine (13).

La garniture (11) a une épaisseur inégale liée à deux rôles essentiels: elle doit amortir au maximum les forces appliquées sur le dispositif une fois mis en place, pour protéger la denture et les éventuelles reconstitutions prothétiques fixes; et elle doit assurer au niveau des bords de la gouttière (1), par compression de la mousse, une sustentation, une stabilité et une rétention similaires à celles qui sont recherchées pour le maintien en place du dentier ou de l'appareil complet maxillaire. C'est pour cela que la garniture (11) recouvre bien les bords de la gouttière (1) tout en s'épaississant pour former un bourrelet périphérique (17); c'est pour cela qu'elle (11) épouse bien la forme de l'échancrure en V de la gouttière (1) tout en recouvrant bien son pourtour; c'est pour cela qu'elle recouvre totalement l'échancrure en V (9) de la gouttière (1) tout en s'amincissant à ce niveau; c'est pour cela qu'au fond de la gouttière, elle s'épaissit légurement dans la zone (18) qui tient lieu de tampon amortisseur vis-à-vis des dents. Celles-ci, quand le dispositif est mis en bouche, sa placent dans l'espace (19) où elles se calent en pénétrant dans la zone (18); dans la zone (20) comprise entre les bourrelets périphériques (17) externe et interne, la gencive se trouve étroitement enserrée.
la rigidité de la gouttière (1), au niveau de la ligne muco-gingivale, associée au joint périphérique étanche réalisé par la compression du bourrelet (17) de la garniture, contribue à assurer au dispositif une stabitité nécessaire et suffisante.
la solidarisation des deux parties, gouttière (1) et garniture (11) est assurée par un adhésif non toxique approprié aux matériaux employés.

La colle est une colle biologique à base de glycosaminoglycannes tels que l'acide hyaluronique ou le chondroïtine sulfate. Elle doit être non allergisante et non irritante pour les muqueuses. Cette colle est déposée sur le bourrelet périphérique (17) de la garniture (11) pour consolider, pendant un temps bref, le maintien en place du dispositif. Le principe du collage est celui du timbre poste, c'est l'humidification de la colle biologique par la salive. Ce collage léger, de courte durée (car la colle se dégrade rapidement) n'est qu'un apport supplémentaire, mais non négligeable, dans le maintien du dispositif.

Pour plus de sécurité, un cordon relie le dispositif placé an bouche à l'extérieur de la cavité buccale. Ce cordon est fixé à un élément extérieur de façon que l'on puisse récupérer le dispositif en cas de chute dans l'oro-pharynx.

Le dispositif existe en trois dimensions correspondant à trois coloris différents. Le praticien sera donc amené à choisir le modèle selon la largeur de l'arcade dentaire qui pourrait être évaluée avec un compas muni de deux pointes mousses. L'appréciation de l'arcade devient, à force d'habitude, une chose banale.

Projetée sur un plan horizontal, la gouttière (1) de taille intermédiaire apparaît comme un fer à cheval qui s'inscrit dans un rectangle dont les dimensions sont de l'ordre 50 mm X 65 mm, la première dimension étant la dimension axiale.

L'épaisseur des parois (6) et (7) de la gouttière (1) est de l'ordre de 1 mm, sauf dans le fond (3) de la gouttière (1) où elle est de 1,5mm à 2 mm.

La distance entre les parois latérales (6) et (7) de la gouttière (1) varie entre 10 et 12 mm.

L'épaisseur de la garniture (11) contre les parois (6) et (7) est de l'ordre de 1 à 2 mm. L'épaisseur du bourrelet périphérique (17) de la garniture (11) qui est de 3 à 4 mm dans la partie antérieure (8), décroît progressivement jusqu'aux extrémités (2) pour atteindre 2 à 3 mm, cela afin de prendre en considération le volume des différentes dents.

Dans la zone (18), l'épaisseur moyenne de la mousse est de l'ordre de 2 mm.

La densité de la garniture (11) doit être choisie de telle sorte qu'elle (11) puisse amortir une pression comprise entre 100 et 150 Kg/cm².

La fabrication du dispositif, tel qu'il vient d'être décrit, peut se faire par emboîtage de la gouttière (1) et de la garniture (11), ces deux éléments étant solidarisés par un adhésif approprié. Ce dispositif peut être obtenu par d'autres techniques, notamment celle qui consiste, à partir d'une pièce brute, à donner la conformation désirée à la garniture par thermo-moulage et, à obtenir par compression ou par l'utilisation de la chaleur ou de tout autre moyen physicochimique ou chimique adéquat, une rigidité suffisante dans les parties interne et externe qui prennent alors les caractéristiques de la gouttière (1).

L'ordre des opérations citées pour la fabrication selon cette autre technique n'est pas impératif. L'ordre des opérations peut être interverti ou les traitements peuvent être concomitants.

La fabrication du dispositif, se fait aussi par thermo-moulage, à partir de matériaux de différentes densités, associés par chauffage, collage ou tout autre procédé d'assemblage avant de subir l'opération de thermo-moulage proprement dite.

Ce dispositif peut avoir d'autres modes d'utilisation. Par exemple, il peut être employé comme gouttière de fluoration dans la prévention des caries dentaires.

## Revendications

1. Dispositif à usage médical destiné à s'adapter aux arcades dentaires pour faciliter l'introduction d'appareillage médical ou chirurgical dans la cavité orale, pour répartir uniformément, sur l'ensemble des arcades dentaires, la pression exercée, dans l'axe des dents, par l'appareillage introduit et aussi pour amortir les forces résultant de la contraction violente exercée par le patient, en présence ou non d'un appareillage médical ou chirurgical entre les arcades dentaires, comprenant une gouttière (1) rigide en forme de fer à cheval qui comporte intérieurement une garniture (11) préformée, caractérisé en ce que la gouttière (1) se termine par un bourrelet périphérique (17) et en ce que le dispositif comporte une zone de moindre résistance constituée par une échancrure (9) en V ou par une zone de plus faible épaisseur, ménagée dans la paroi interne (6) de la gouttière (1) et qu'il présente une échancrure (10) en U, ménagée dans la paroi externe (7) de la gouttière (1).

2. Dispositif selon la revendication 1, caractérisé en ce que le bourrelet périphérique (17) de la garniture (11) qui prolonge les parois interne (6) et externe (7) est conçu de telle sorte qu'il joue le rôle d'un crampon.

3. Dispositif selon les revendications 1 et 2, caractérisé en ce que la garniture préformée (11) présente dans le fond (3) de la gouttière (1) une zone (18) à épaisseur plus grande que celle réalisée au niveau de l'échancrure en V (9), la zone (18) étant destinée à jouer le rôle d'un tampon-amortisseur dans l'axe des dents.

4. Dispositif selon les revendications 1, 2 et 3, caractérisé en ce qu'une colle biologique, composée à base de glycosaminoglycannes est déposée sur le bourrelet périphérique (17).

5. Dispositif selon les revendications 1, 2, 3 et 4, caractérisé en ce que la garniture (11) est réalisée en un matériau à cellules fermées: le polyéthylène expansé à l'azote pur.

## Claims

1. Medical device for maxillary dental arches used to facilitate the introduction of a medical or surgical equipment into the oral cavity in order to distribute uniformly on the whole dental arches the pressure provided in the axis of the teeth by the introduced equipment and also to damp the forces resulting from the high contraction exerted by the patient with the presence or not of a medical or surgical equipment between the dental arches, comprising a rigid horseshoe gutter (1) having inwardly a preformed packing (11), characterized in that the gutter (1) ends with a circonferential pad (17) and in that the device comprises a lower resistance area having a V notch (9) or a smaller thickness area provided in the internal wall (6) of the gutter (1) and a U notch (10) provided in the external wall (7) of the gutter (1).

2. Device according to claim 1, characterized in that the circonferential pad (17) of the packing (11) extending the inner wall (6) and the external wall (7) is made so as to act as a clamp.

3. Device according to one of claims 1 and 2, characterized in that the preformed packing (11) comprises in the bottom (3) of the gutter (1) an area (18) of greater thickness than the one provided at the level of the V notch (9), the area (18) acting as a damp pad in the axis of the teeth.

4. Device according to one of claims 1, 2, 3, characterized in that a biological glue comprising glycosaminoglycannes is laid on the circonferential pad (17).

5. Device according to one of claims 1, 2, 3 and 4, characterized in that the packing (11) is made with a closed cell material : polyethylene expanded with pure nitrogen.

## Patentansprüche

1. Vorrichtung für medizinische Zwecke, die sich zur Erleichterung des Einführens medizinischer oder chirurgischer Vorrichtungen in den Mundhohlraum an die Zahnbögen anpaßt, um den von der eingeführten Vorrichtung aufgebrachten Druck gleichmäßig in Achsrichtung der Zähne über die Gesamtheit der Zahnbögen zu verteilen, und um die aus der vom Patienten ausgeübten starken Kontraktion resultierenden Kräfte zu dämpfen, unabhängig vom Vorhandensein oder Nichtvorhandensein einer medizinischen oder chirurgischen Vorrichtung zwischen den Zahnbögen, mit einer hufeisenförmigen starren Rinne (1), die auf der Innenseite mit einer vorgeformten Auskleidung (11) versehen ist, dadurch gekennzeichnet, daß die Rinne (1) in einem Randwulst (17) endet, und daß die Vorrichtung eine Zone geringerer Festigkeit aufweist, die durch eine in der Innenwand (6) der Rinne (1) ausgebildete V-förmige Aussparung (9) oder eine Zone geringerer Dicke gebildet ist, und daß die Vorrichtung eine in der Außenwand (7) der Rinne (1) ausgebildete U-förmige Ausnehmung (10) aufweist.

2. Vorrichtung nach Anspruch 1, dadurch gekennzeichnet, daß der Randwulst (17) der Auskleidung (11), der die Innen- (6) und die Außenwand (7) verlängert, derart ausgebildet ist, daß er als Klammer wirkt.

3. Vorrichtung nach den Ansprüchen 1 und 2, dadurch gekennzeichnet, daß die vorgeformte Auskleidung (11) am Boden (3) der Rinne (1) eine Zone (18) aufweist, deren Dicke größer ist als auf der Höhe der V-förmigen Aussparung (9) vorgesehen, wobei die Zone (18) als Stoßdämpfer in der Zahnachse wirkt.

4. Vorrichtung nach den Ansprüchen 1, 2 und 3, dadurch gekennzeichnet, daß ein biologischer Kleber auf der Basis von Glycosaminoglycanen auf dem Randwulst (17) aufgebracht ist.

5. Vorrichtung nach den Ansprüchen 1, 2, 3 und 4, dadurch gekennzeichnet, daß die Auskleidung (11) aus einem geschlossenzelligen Material gebildet ist, nämlich aus mit reinem Sauerstoff geschäumtem Polyethylen.
